# EUROPEAN PATENT APPLICATION

(11) **EP 4 303 324 A1**
(43) Date of publication of application: **10.01.2024**
(21) Application number: 22460031.2
(22) Date of filing: 05.07.2022
(51) Int. Cl.: C12Q 1/6886

(54) **A METHOD OF DISTINGUISHING BETWEEN BENIGN AND MALIGNANT THYROID NODULES**

(71) Applicant: Narodowy Instytut Onkologii im. Marii Sklodowskiej-Curie Panstwowy Instytut Oddzial w Gliwicach, 44-102 Gliwice (PL); Politechnika Slaska, 44-100 Gliwice (PL); Polskie Towarzystwo Endokrynologiczne, 02-097 Warszawa (PL); Uniwersytet Medyczny w Lodzi, 90-419 Lodz (PL); WASKO Spólka Akcyjna (WASKO S.A.), 44-100 Gliwice (PL)
(72) Inventor: Krajewska, Jolanta, 44-100 Gliwice (PL); Pluciennik, Alicja, 44-100 Gliwice (PL); Oczko-Wojciechowska, Malgorzata, 41-808 Zabrze (PL); Placzek, Aleksander, 44-100 Gliwice (PL); Jarzab, Michal, 41-807 Zabrze (PL); Student, Sebastian, 44-100 Gliwice (PL); Wilk, Agata, 44-100 Gliwice (PL); Czarniecka, Agnieszka, 44-100 Gliwice (PL); Pfeifer, Aleksandra, 41-705 Ruda Slaska (PL); Kotecka-Blicharz, Agnieszka, 44-100 Gliwice (PL); Lakomiec, Krzysztof, 44-100 Gliwice (PL); Chmielik, Ewa, 44-100 Gliwice (PL); Slowinska-Klencka, Dorota, 90-419 Lódz (PL); Dedecjus, Marek, 02-781 Warszawa (PL); Jarzab, Barbara, 44-101 Gliwice (PL); Fujarewicz, Krzysztof, 43-100 Tychy (PL)

(57) **Abstract**

A method of distinguishing benign and malignant thyroid nodules in the nodule biopsy sample, classified according to the Bethesda system, including the steps of RNA isolation and the determination of expression level of a normalized gene set, including AMOT, DCSTAMP, NOD1, SLC26A7, EMP2, EGR1, MPZL2, ITGA2, MET, SLPI, TPO. To differentiate thyroid nodules, a classifier algorithm is used, which is trained on gene expression data correlated with the nature of the thyroid nodule. In addition, the probability of malignancy of the test sample is determined.

## Description

The invention relates to a method of distinguishing benign and malignant thyroid nodules and is intended for use in the area of preoperative diagnosis.

For the purpose of implementing the invention, a classifier with a set of classifying features is also presented, and the evaluation of expression of a set of genes can be used for both papillary and follicular carcinomas, which are collectively referred to as differentiated thyroid carcinomas.

Existing methods of distinguishing thyroid nodules do not provide complete safety in assessing that we are dealing with a benign thyroid nodule, which can be left without surgery without any harm to the patient.

The purpose of the present invention is to provide a more effective way to distinguish between malignant and benign thyroid nodules.

The normal follicular thyroid cell is highly differentiated, showing specialized features. Abnormal proliferation of thyroid cells can result in the formation of nodules, which can be benign or malignant. Malignant thyroid tumors come in three main forms: differentiated carcinomas, medullary carcinoma, and anaplastic carcinoma. Papillary thyroid carcinoma (PTC) is the most common type of differentiated thyroid cancer originating from the follicular thyroid cell. Diagnosis of follicular thyroid carcinoma (FTC) poses the most significant diagnostic challenges preoperatively.

Routine diagnosis of thyroid nodules is based on imaging evaluation by ultrasonography and cytological evaluation of material (biological sample) obtained by fine-needle aspiration biopsy (FNAB). The material (biological sample) includes tissue fragments taken from the tumor by puncture and/or aspiration.

The ultrasound classification EU-TIRADS (The European Thyroid Imaging Reporting and Data System) is based on the evaluation of thyroid nodules described by sonographic features and provides five categories of malignancy risk. The imaging evaluation of lesions refers, among others, to echogenicity, shape and margin of the tumor, presence of macro-and microcalcifications, clarity of margins, structure, and vascularization.

Currently, the cytological examination results are used to determine further clinical management. Surgical treatment is required when the result suggests the suspicion of a malignant lesion, while follow-up is usually recommended when a benign lesion is diagnosed. Cytological evaluation is based on the Bethesda system (TBSRTC - The Bethesda System for Reporting Thyroid Cytology), distinguishing six categories from non-diagnostic lesions - B1 to malignant neoplasm - B6, where category B2 corresponds to a benign lesion, B3 - a follicular lesion of undetermined significance, category B4 - suspicious for a follicular neoplasm, and B5 suspicious for malignancy.

Fine-needle aspiration biopsy (FNAB) is the standard diagnostic procedure for suspicious thyroid nodules, but it has many limitations due to its inability to assess the arrangement of follicular cells within the thyroid follicle structure and to evaluate any invasive features. The amount of specimens obtained from the fine-needle biopsy is small, so immunohistochemical methods are difficult to apply. For this reason, nearly 30% of all biopsies of thyroid nodules are classified as indeterminate lesions (category B3, B4, and B5) as unequivocal distinguishing based on FNAB between benign and malignant nodules is not possible. Therefore, most of these patients are now referred for surgical treatment - diagnostic surgery, while cancer is diagnosed postoperatively in only 15-30% of them. It means that most of the resected nodules are benign and do not require such radical medical management. Thus, the thyroid surgery performed may be ex-post considered unnecessary.

Assays to support thyroid cytology, including molecular diagnostics, could influence the reduction of a number of diagnostic surgeries, resulting in a better quality of life for patients and less burden on the health care system. However, it is unclear how much gene-level analysis can approximate the diagnosis estimation, as it may not capture the full differences between the genomes of malignant and benign thyroid nodules.

For example, in US2007037186A1, Jiang et al. revealed a 4-gene QRT-PCR test with a sensitivity of 92% for cancer diagnosis but a specificity of only 61%.

Studies on molecular alterations that play a role in the development of thyroid cancer have established that a point mutation V600E in the BRAF gene, leading to constitutive activation of this serine/threonine kinase, is the most common mutation in thyroid cancer, observed in about 45% of papillary thyroid carcinomas. RAS mutations, also involved in the development of thyroid tumors, are frequently found in follicular adenomas, suggesting their potential role in early follicular cell oncogenesis. Mutations of PTEN, PIK3CA, and other genes, as well as gene translocations (RET-PTC, PAX8-PPARG) and abnormal gene methylation, are considered critical molecular alterations. However, using some of these molecular markers in preoperative diagnosis is still insufficient to distinguish benign from malignant tumors with adequate sensitivity and specificity.

Gene classifiers based on assessing mutations of a few or even dozens of genes or evaluating the expression of a set of genes are well known. The most widespread is the expression classifier *(Afirma Gene Expression Classifier; Veracyte Inc; USA),* based on the evaluation of the expression of a gene set using high-density oligonucleotide microarrays. It is a "rule out malignancy" test, and the result is primarily used to exclude malignancy of the nodule.

The latest version of the test, the Genomic Sequencing Classifier (GSC), assesses gene expression and mutations. The test allows the analysis of several thousand genes, including detecting changes originating from Hürthle cells.

There are also known tests based solely on tumor mutation (DNA) analysis and similar tests with additional microRNA (miRNA) expression analysis to obtain high sensitivity and specificity.

There are no validation tests involving the European population for the abovementioned gene classifiers.

Known from the description of the EP3119886B1 patent is a copy number-preserving method of RNA analysis, in particular, analyses for estimating the amount and type of transcript, and a kit for performing it, which may contain components suitable for carrying out the method according to their function.

The invention, known from the EP2505664B1 patent description, relates to a method for distinguishing between malignant and benign thyroid nodules by performing RNA extraction in a standard FNAB specimen, particularly in an air-dried FNAB smear. The isolated RNA is analyzed for the presence of gene rearrangements and/or miRNA expression, with the presence of RET/PTC and/or PAX8/PPARG rearrangements and/or mutations in genes encoding BRAF, N-, K-, and/or HRAS and/or differential miRNA expression in the sample classifying the tumor as malignant.

The use of differentially expressed genes in benign and malignant thyroid lesions for the diagnosis and staging of thyroid cancer is revealed in the description of the EP1756303B1 patent. In turn, the description of the US8541170B2 patent includes compositions, sets, and methods for molecular profiling and cancer diagnosis, including markers for gene expression products, alternative markers for exon usage, and cancer-associated DNA polymorphisms. Similarly, the description of the US7901888B2 patent provides information on ways to diagnose, predict and stage thyroid cancer using molecular markers.

The system for classifying a thyroid nodule as malignant or benign, known from the description of the US9617604B2 patent, is based on identifying sets of gene transcripts and involves determining the expression level of one or more transcripts in a sample, at least one of which contains the corresponding at least one option from SEQ ID Nos. 1-12, 261, 283-306, 657, 658 and 659. In addition, the classification system provides sets of target sequences and combinations of polynucleotide probes and derived primers that can be provided in a solution or an array.

The description of the US9708667B2 patent provides a method for classifying thyroid tumors by analyzing the expression patterns of specific microRNAs extracted from a thyroid sample taken by fine-needle aspiration and classified as category III, IV, or V according to the Bethesda system. The expression levels of miRNAs are then determined, and thyroid lesions are classified as benign or malignant based on the results of the classifier algorithm. The US10236078B2 and US10672504B2 patent descriptions relate to methods for molecular profiling and diagnosis of genetic disorders and cancer, including gene expression markers associated with cancer or genetic diseases. Both inventions provide algorithms and methods for classifying thyroid cancer, determining molecular profiles, and analyzing the results to make a diagnosis.

The US10260103B2 patent relates to diagnostic tests for identifying thyroid cancer in a biological sample. It further discloses a method for diagnosing thyroid cancer based on determining the expression levels of three or more genes of a thyroid tissue sample, wherein at least one of the gene products is expressed by the CXCR3, CCR3, CXCL10, CAR, XB130, HO-1, or CCR7 gene.

A method for diagnosing a genetic disorder or cancer as known from the US20202974A patent includes detecting gene expression in a biological sample, comparing gene expression in the biological sample with a control sample, classifying the biological sample by inputting one or more differential gene expression levels into a trained algorithm; and identifying a biological sample as positive for a genetic disorder or cancer if the trained algorithm classifies the sample as positive for a genetic disorder or cancer at a certain level of specificity and/or sensitivity.

The invention, according to the EP2569626B1 patent, relates to components, kits, and methods of molecular profiling for diagnosing thyroid nodules. The method of evaluating a thyroid nodule sample involves determining the expression level of one or more genes, one or more of which correspond to at least five genes selected from SNCA, STK32A, THRSP, TIMP1, TIMP2, TMSB10, TNFRSF17, TNFRSF1A, TXNDC12, VWA5A, WAS, WIPI1 and ZFYVE16 and then classifying the thyroid nodule as benign or suspicious by comparing the gene expression levels of the sample with gene expression data for at least two different sets of biomarkers. Gene expression data for each set of biomarkers includes one or more reference gene expression levels correlated with the presence of one or more tissue types. The expression level is compared sequentially with gene expression data for the mentioned at least two different sets of biomarkers.

According to the EP2925886B1 patent, a thyroid tissue sample is identified as malignant if the expression level of any one or more of the CXCR3, CAR, and/or XB130 genes is decreased; and/or the expression level of any one or more of the CCR3, CXCL10, CK-19, TIMP-1, CLDN-1, and/or CCR7 genes is increased in the thyroid tissue sample as compared to the normal control expression level, wherein the total number of genes with increased or decreased expression is at least three.

Unless otherwise specified, all technical and scientific terms used herein shall have the same meaning as commonly understood by a person of average skill in the field to which this invention belongs.

Terms used in the patent description and claims have the following meanings:
- **Thyroid nodule** - any focal lesion in the thyroid gland, benign or malignant, which can be subjected to the diagnostic process for definitive evaluation of the nature and clinical aggressiveness;
- **Gene expression** - the process by which the genetic information contained in a gene is read and transcribed into its products in the form of mRNA
- **Gene expression profile** - This term stands for a set of qualitative and quantitative information regarding the readout of a set of genes in a cell, which indicates their activity. It refers to all or selected gene sets identifiable for a particular type of tissue or cell.
- **Training data** - the dataset used to determine and validate the parameters of the prediction model;
- **Reference gene, control gene, normalizing gene** - a gene whose expression is maintained at a constant level in normal and neoplastic cells regardless of their nature and phenotype
- **Thyroid nodule sample** (interchangeable with: tumor material, biological sample or specimen) - fresh and frozen samples, stored in liquid nitrogen or dry ice, and samples fixed with fixatives; taken by the FNAB method. The sample contains thyroid cells;
- **qPCR** (Quantitative polymerase chain reaction) - a real-time polymerase chain reaction that allows the assessment of transcript expression
- **Ct (cycle threshold)** - The reaction cycle at which the fluorescence of a sample exceeds a cut-off line (threshold), set automatically or by the operator. Ct is measured in a logarithmic increase in the amount of DNA, and the smaller its value is the higher the concentration of array was at the beginning of the reaction.
- **DNA microarray** - a plate with applied probes that allow assessment of the expression of many thousands of transcripts simultaneously, approximately determines the expression of the entire transcriptome of a given cell.

The method of distinguishing benign and malignant thyroid nodules in a tumor biopsy sample classified according to the Bethesda system from II to VI, according to the invention, includes the steps of RNA isolation and determination of the expression level for a larger number of genes. A comparison of the gene expression of the thyroid nodule sample with the gene expression of the samples in the training dataset is conducted using the classifier algorithm, and next, the sample is identified as malignant or benign, and the probability of malignancy of the thyroid nodule is determined.

The classifier algorithm with a set of classifier features is taught on a training dataset of samples of thyroid nodules with gene expression correlated with the nature of the thyroid nodule, especially benign nodules or malignant tumors, including follicular thyroid carcinoma and papillary thyroid carcinoma.

The method is characterized by that the expression level of a set of the following genes DCSTAMP, NOD1, SLC26A7, EMP2, EGR1, MPZL2, ITGA2, MET, SLPI, and TPO is determined and the normalization of the expression of each gene of this set is carried out under the endogenous control of at least four normalizing genes among the following genes: ElF3A (EIF3S10), HADHA, UBE2D2, EIF5, PPIE, and RPLP0, most favorably, these six normalizing genes.

A normalized expression level of a set of genes above or below a certain differential level indicates the malignant nature of the nodule, with at least 90% accuracy of the thyroid nodule malignancy diagnosis.

The probability of malignancy of a tested sample is determined according to the classifier's decision rule on the basis of classifier features taking into account the gene expression levels in the training set, determined for each sample of this set for the same set of genes and under the same normalization conditions.

For each sample of the training dataset, the set of classifying features includes at least one among: cytological evaluation, nodule size, age, and sex of the patient, expressed in numerical form or as the value of a transforming function.

RT-qPCR, oligonucleotide microarray, or RNA-seq transcriptome sequencing methods are used to detect the expression levels of a set of genes.

The invention also relates to the use of a set of hybridization probes provided in Table 3, columns 2 and 6 for detecting in a thyroid nodule sample the expression level of a corresponding gene from a set of 10 genes, as well as for determining the nature of the nodule by the method according to claim. 1, using a computer software product having computer-readable storage device and compositions for measuring the normalized expression level of a set of genes, performing classification and determining the probability of the malignancy of the nodule sample.

The method, according to the invention, uses molecular features, including a small number of genes and phenotypic features, to assess the probability of malignancy of the tested sample. It facilitates the interpretation of the results and has an impact on improving the quality of classification, which enables decision-making regarding further treatment. The small number of genes and the use of a single method to evaluate all the features tested influence the low cost of the test, which is important for health care systems. In addition, it provides the result of the test in a short period of time.

The method, according to the invention and example test results, are illustrated in the following figures:
Fig.1 and Fig.2 show a plot of quality parameter values for different threshold values for the [B26] and [B345] classifier models, respectively.
Fig.3 and Fig.4 show the classifier's quality characteristics as a ROC curve for the tested samples belonging to the Bethesda B2 and B6 categories (Fig.3) and the Bethesda B3, B4, and B5 categories (Fig.4), respectively
Fig.5 to Fig.8 show box plots of the results of differentiating the thyroid nodule samples tested, as described in Examples 2 to 5. The individual figures show the gene expression distributions for the training population and the result of the test against their background.

They also indicate the nature of the tested nodule sample and the result of the probability of malignancy, which was determined with its appropriate classifying features as the input parameters.

Ultrasound examination is the first step in the diagnosis of a thyroid nodule after clinical evaluation. Depending on the estimated risk for a given image, the threshold size of the thyroid nodule for further evaluation is determined. A fine-needle aspiration biopsy of a thyroid nodule provides material for cytologic analysis. Based on this, the nodule is assigned to a specific Bethesda category.

The cytological evaluation of a nodule sample is a subjective assessment based on the physician's professional experience. In about 30% of biopsies, cytologic evaluation (indeterminate thyroid nodule) does not allow a clear exclusion of malignancy. Indeterminate nodules, i.e., those in which the result of the cytologic evaluation may be questionable, are the subject of preoperative molecular diagnostics.

The differentiation of a thyroid nodule as benign or malignant is based on multidimensional analysis of a fine-needle biopsy sample of the nodule containing RNA isolation material using an appropriate classifier based on classifying features, including gene expression values.

For the purpose of implementing the invention, the computer system provides a product in the form of computer software, having a computer-readable storage device and the resources to carry out classification, modeling, and gene expression measurement methods - as an expression classifier (hereinafter classifier). For this purpose, it has at least one processor and at least one program memory, which contains the processor's execution instructions (classifier algorithm) for analyzing data and determining gene expression levels. Based on the classifier algorithm, decision rules are generated. In addition, it contains datasets relating to the evaluation of fine-needle biopsy samples of thyroid nodules. The information concerning thyroid nodules includes age, sex, lesion size, shape, Bethesda categories, multifocality, echogenicity, and additional information on involved lymph nodes. The classifier as a predictive model can distinguish and characterize samples based on established characteristics. For this purpose, it can apply appropriate rules to filter sample data.

To calculate the probability of malignancy from SVM models, it is possible to use probability scaling methods, such as the Vapnik scaling method, isotonic regression, or Platt scaling. The SVM model, according to the example implementation of the invention, uses the Platt method, based on the maximum likelihood, to calculate the probability of malignancy of a sample.

Platt scaling transforms the results of the SVM classifier into a probabilistic model under which the data values are most likely. The evaluation of the probability of malignancy is based on training data.

The probability of malignancy of a tested sample is determined using a probabilistic model based on the sample's classification characteristics.

Each sample is identified by ultrasound and cytologic results, as well as molecular results based on gene expression evaluation. The classifier consists of two SVM models, including samples with Bethesda categories established by cytological examination. The classifier features, in turn, take into account the results of the thyroid nodule sample from cytological and molecular studies, expressed in numerical form or as the value of a transforming function. This allows for an integrated assessment of cancer risk.

For the research phase, more than 3,000 samples from fine-needle biopsies of thyroid nodules were collected. One or two additional samples were collected from each patient to obtain sufficient material to isolate nucleic acids for molecular analysis. Total RNA was used in the study. Material collected by aspiration is protected in preservation buffer, frozen, and then RNA isolation is performed.

In addition, an analysis of 16,000 retrospective data obtained from cytological examinations of the thyroid nodules with Bethesda category ranging between B2 - B6, diagnosed between 2007 and 2017, based on the Polish population, confirmed the statistical representativeness of the data of the training and validation sets presented below.

Based on the clinical parameters of the nodule, cytological examination data, and gene expression data, a diagnostic test is carried out. Gene expression analysis is carried out by qPCR, microarray, or NGS methods.

For the implementation of the first stage of the development of the expression classifier, the material was collected for the training set. The training set comprised 336 thyroid nodule samples, including 70 nodules with a cytologic diagnosis of a benign lesion (Bethesda II - B2), 68 nodules with a cytologic diagnosis of follicular lesion of undetermined significance (Bethesda III - B3), 61 nodules with cytologic diagnosis suspicious for a follicular neoplasm (Bethesda IV - B4), 47 nodules suspicious for thyroid cancer (Bethesda V - B5) and 90 nodules with a cytologic diagnosis of thyroid cancer (Bethesda VI - B6) - the data are given in the table below.

| | **The whole training set** | **Benign nodules** | **Malignant nodules** |
|---|---|---|---|
| **Number** | **336** | **207** | **129** |
| **F/M** | **283152** | **173/30** | **107/22** |
| **Median age** | **57** | | |
| **Symptoms** | **27/336** | **14/207** | **131129** |
| **Median nodule diameter** | **15** | | |
| **EU TIRADS 3** | **991328** | **78/201** | **21/127** |
| **EU TIRADS 4** | **102/328** | **81/1201** | **21/127** |
| **EU TIRADS 5** | **127/328** | **42/201** | **85/127** |
| **Bethesda 11** | **70/336** | **70/207** | **0/129** |
| **Bethesda III** | **68/336** | **65/207** | **3/129** |
| **Bethesda IV** | **61/336** | **56/207** | **5/129** |
| **Bethesda V** | **47/336** | **16/207** | **31/129** |
| **Bethesda VI** | **90/336** | **0/207** | **90/129** |

High-density microarray Human Transcriptome 2.0 (Affymetrix) was used to analyze the gene expression profile.

After preprocessing and background correction of the entire microarray set, the microarray set underwent a normalization process. To define the probe sets representing genes from the ENTREZ database, for all microarrays, a custom CDF (Chip Definition File) version 21 was used, which was downloaded from the website:
http://brainarray.mbni.med.umich.edu/Brainarray/Database/CustomCDF/CDF_download.asp The clinical analysis included 174 patients with a Bethesda II cytological diagnosis, 119 with a Bethesda III diagnosis, 126 with a Bethesda IV diagnosis, 76 with the Bethesda V category, and 104 with the Bethesda VI category.

In the Bethesda II category group, ten patients were operated on with no diagnosis of thyroid cancer. In the Bethesda III group, 55 patients were operated on, diagnosing thyroid cancer in 11 cases (1 patient medullary thyroid carcinoma, 10 patients follicular thyroid carcinoma). In the Bethesda IV group, 85 patients were operated on and thyroid cancer was confirmed in 17 cases (4 patients papillary thyroid carcinoma, 7 patients follicular thyroid carcinoma, 3 patients oncocytic thyroid carcinoma, 3 patients poorly differentiated thyroid carcinoma). In the Bethesda V group, 75 patients were operated on, diagnosing thyroid cancer in 63 cases (61 patients with papillary thyroid carcinoma and 2 patients with medullary thyroid carcinoma). In the Bethesda VI group, all patients underwent surgical treatment. Thyroid cancer was diagnosed in 101 cases (99 patients - papillary thyroid carcinoma, 1 patient follicular thyroid carcinoma, 1 patient poorly differentiated carcinoma).

The clinical analysis was followed by testing the classifier model developed on the basis of the training set. Samples with poorly differentiated thyroid cancer, medullary thyroid cancer, and samples of questionable quality were excluded from the analysis.

Developed tool using expression of a set of genes DCSTAMP, NOD1, SLC26A7, EMP2, EGR1, MPZL2, ITGA2, MET, SLPI, and TPO is diagnostically effective. 93% of the tested thyroid nodules were correctly classified as benign or malignant. The estimated sensitivity and specificity of the diagnostic test were about 95% and 85%, respectively.

In the next step, the material for the validation set was collected, RNA was isolated for the selected samples from the validation set, quantitative PCR was performed for the selected samples, and clinical analysis was performed.

To test the classifier model, 462 samples including 58 thyroid nodules classified by cytological evaluation as B2, 85 B3 nodules, 111 B4 nodules, 98 B5 nodules, and 120 B6 nodules were used.

Correct results consistent with the histopathological diagnosis were obtained in 408 nodules, whereas 54 remaining nodules were misclassified.

Next, the samples erroneously evaluated by the classifier were analyzed, finding no substantive errors and errors that may be due to the mistakenly assigned nature of the sample.

The classifier (as a predictive model) operates on datasets of fine-needle biopsy samples of thyroid nodules and an associated multivariate set of descriptor variables, including gene expression profile. The samples used for training are a part of the model. Based on specific sample characteristics, it can therefore distinguish or characterize samples and classify a given sample according to those features. The classifier makes it possible to predict the class of a sample and determine the sample's malignancy probability based on a radial kernel SVM model, trained on a training set, and validated using a bootstrap method (500 iterations). Based on the estimated risk of malignancy determined by the SVM model, using the Platt method, based on the criterion of the maximum likelihood to calculate the probability of malignancy, a given nodule is classified as a benign or malignant lesion based on an optimal threshold.

There are two models within the classifier, and the choice of model depends on the cytological evaluation according to Bethesda categories.

The first classifier model [B26] is taught on a training set containing samples from the BII and BVI categories and is designed to predict their risk of malignancy, with a default cutoff according to the "malignant" class (tumor malignancy) = 0.5.

While, the second classifier model [B345] has an assigned cytological evaluation of samples in the Bethesda III, IV, and V category and is designed to predict their malignancy risk. The model is taught on a full training set (B2-B6), and the default cutoff by "malignant" class (tumor malignancy) = 0.53.

The numerically presented Bethesda category strengthens the indication of the result obtained by the classifier if there is a conflict between clinical and molecular information. An example is a follicular carcinoma, which cannot be distinguished from benign follicular adenoma by cytologic examination. Due to the possible classification of this case as the Bethesda B2 category, according to the current rules in other solutions, it would not be subjected to molecular testing. On the contrary, the present invention incorporates genomic and clinical features into a single classifier, enabling integrated cancer risk assessment.

For the training set, 336 samples were collected from thyroid nodule biopsies of patients who underwent surgical treatment, and thyroid cancer was excluded or confirmed by histopathological examination. The cytological examination was performed, and the result was determined according to Bethesda categories. Isolation of mRNA was performed, and the expression of a set of 10 genes was measured using the microarray method.

For 282 samples from the training set, the obtained RNA concentrations ranged between 4 ng/µl up to 200 ng/µl with a median of 16,7 ng/µl. For 160 samples, RNA concentration was below <20 ng/µl.

Classifier models using the expression of a set of genes and the cytological assessment, expressed numerically, as classifier features were applied. The postoperative histopathological result was used to determine the quality of the applied classifier for the tested set of samples. The characteristics of the predictive ability of the classifier models [B26] or and [B345] are presented below in a tabular overview.

| **Model** | **Accuracy [CI 95%]** | **Sensitivity** | **Specificity** | **NPV** | **PPV** |
|---|---|---|---|---|---|
| [B26] | 0.98 [0.96, 1.0] | 1 | 0.97 | 1 | 0.98 |
| [B345] | 0.90 [0.84, 0.95] | 0.68 | 0.95 | 0.91 | 0.82 |

The obtained high values of the tested parameters of the classifier models [B26] and [B345] confirm their usefulness for distinguishing the collected samples before the decision on surgery or its extent (total or partial thyroidectomy) or for verifying the decision to perform surgery already made.

The cutoff threshold used makes it possible to control the sensitivity and specificity of the test, including the PPV (positive predictive value) and NPV (negative predictive value) parameters. We investigated how the quality parameters change when the threshold changes in the function determining the classification label (benign/malignant) in the range of values 0.4 - 0.6. The analysis was carried out taking into account the changes in thresholds by different classifier models [B26] and [B345].

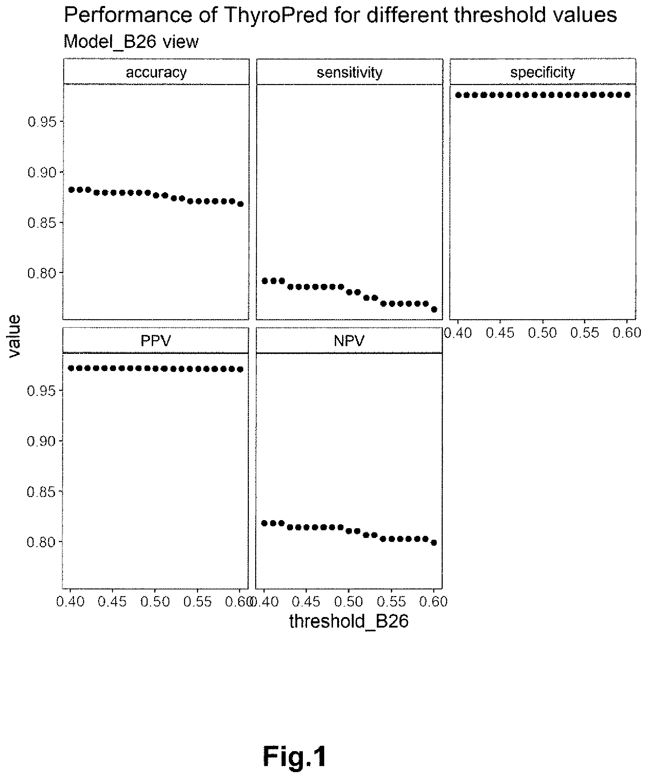

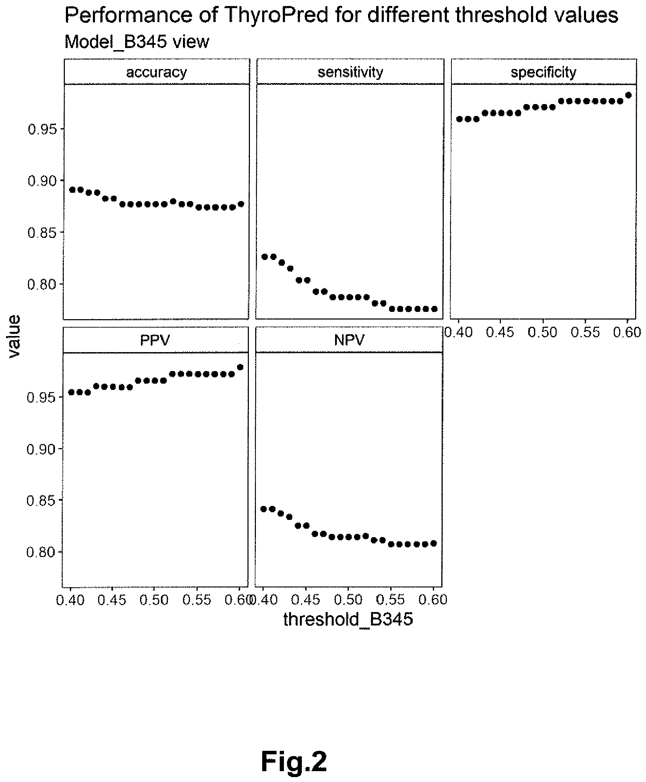

The results, shown in the graphs in Fig.1 and Fig.2, respectively, reveal the relationships between the different parameter groups, where the classifier can be optimized against either PPV or NPV for models [B26] and [B345], respectively. Optimizing a classifier against PPV increases its specificity, and against NPV increases its sensitivity or accuracy.

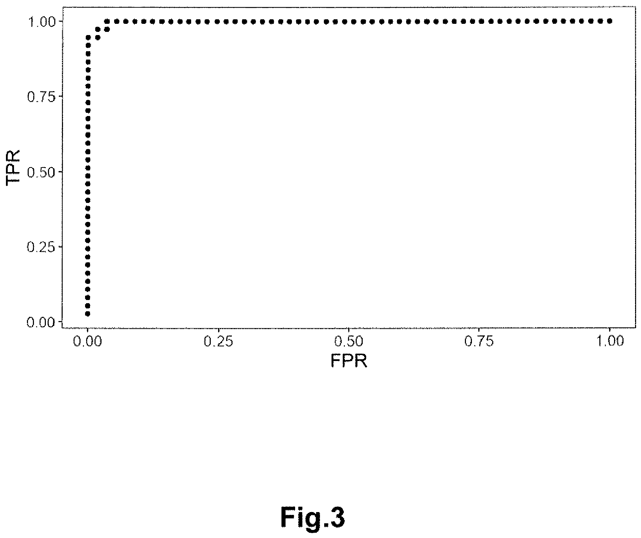

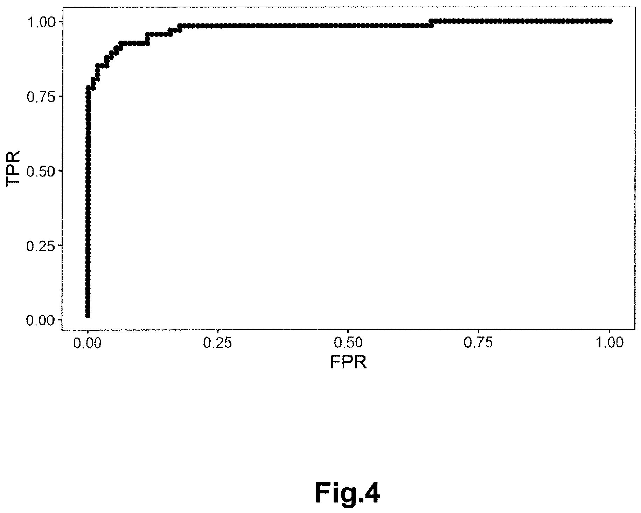

The highest classification accuracy is obtained if the threshold of the classifier function is as low as possible in both models. In contrast, the highest PPV has a classifier with a high threshold for the B345 classifier model but it has lower classification accuracy and sensitivity. The classifier with the lowest thresholds in both models has the highest NPV - classification accuracy is also highest for this setting. Fig.3 displays the characteristics of the classifier's quality presented as a ROC curve for the tested samples [826]. Fig.4 displays the ROC curve for the classifier [B345]. The ROC curve shows the characteristics of the classifier's sensitivity and specificity. The shape of the ROC curve indicates that the classifier correctly assigns labels benign/malignant.

In the context of interpreting the settings of the classifier, where the positive class was assumed to be malignant neoplasm - a high PPV indicates the probability of correctly detecting features of malignant neoplasm. In contrast, a high NPV indicates the probability of correctly detecting features of a benign lesion (excluding a malignant lesion). The differential level determined above indicates the malignant nature of the thyroid nodule, with at least 90% accuracy

A bootstrap method was used to evaluate the classification quality of feature models for variants and combinations of feature selection methods, data fusion, and classification algorithms. The selection and classification algorithms with the best classification quality parameters were selected. Based on them, a set of genes was selected for the RT-qPCR - Openarray plate. This set involves among others the following genes DCSTAMP, NOD1, SLC26A7, EMP2, EGR1, MPZL2, ITGA2, MET, SLPI, and TPO shown in the table 1. Extended information on the set of genes is provided in Table 3.

**Table 1**

| **Lp** | **Gene name** | **ENTREZ ID** | **Assay ID** | **RefSeq(s)** | **Function** |
|---|---|---|---|---|---|
| **1** | **DCSTAMP** | 81501 | Hs00229255_m1 | XM_017013878.1; | Analyzed gene |
| | | | | XM_011517321.1; NM_030788.3 | |
| **2** | **NOD1** | 10392 | Hs01036720_m1 | XM_011515079.1; M_005249572.1; NM_006092.2; XM_005249568.1; XM_011515088.2;XM_011515087.1 XM_017011674.1;XM_017011675.1 XM_011515085.1;XM_011515084.1 XM_011515083.1;XM_006715633.2 XM_011515081.2;XM_005249576.1 XM_011515080.2 | Analyzed gene |
| **3** | **SLC26A7** | 115111 | Hs01104163_m1 | NM_052832.3;NM_001282357.1; NM_001282356.1;NM_134266.1 | Analyzed gene |
| **4** | **EMP2** | 2013 | Hs00171315_m1 | NM_001424.5;XM_006720864.3 | Analyzed gene |
| **5** | **EGR1** | 1958 | Hs00152928_m1 | NM_001964.2 | Analyzed gene |
| **6** | **MPZL2** | 10205 | Hs01083647_m1 | NM_144765.2;NM_005797.3 | Analyzed gene |
| **7** | **ITGA2** | 3673 | Hs00158127_m1 | NM_002203.3;NR_073103.1; NR_073104.1;NR_073105.1; NR_073106.1 | Analyzed gene |
| **8** | **MET** | 4233 | Hs01565584_m1 | XM_006715990.2;NM _001127500.2; NM_001324401.1;NM_001324402.1 XM_011516223.1;NM_000245.3 | Analyzed gene |
| **9** | **SLPI** | 6590 | Hs00268204 _m1 | NM_003064.3 | Analyzed gene |
| **10** | **TPO** | 7173 | Hs00892519_m1 | NM_175719.3;NM_175721.3;NM_17 5722.3;NM _001206744.1; NM_001206745.1;NM_000547.5; XM_011510380.2; XM_011510381.2;XM_011510379.2 | Analyzed gene |

The classification test includes a stage of normalization of samples. For proper validation of feature selection, an important part is the selection of reference genes.

The basis of the analysis was to evaluate not only individual genes as candidates for reference genes but also groups of genes capable of performing these functions together.

Because of the importance for the correctness of the result, the normalization process is carried out using normalizing genes with stable expression levels between samples and between calibrator sample plates. A biological material called a calibrator is used to normalize the data between test runs.

Reference gene selection was performed using iterative exclusion of the least stable genes (e.g. the NormFinder method). The set of reference genes includes EIF5, PPIE, UBE2D2, HADHA, EIF3A (EIF3S10), and RPLPO, (Table 2).

It is assumed that each sample should show some minimum expression for at least four reference genes, most favorably of these six normalizing genes. It is claimed that in QPCR reaction a Ct below 28 indicate the sufficient signal of expressed gene.

**Table 2**

| **L.p.** | **Gene name** | **ENTREZ ID** | **Assay ID** | **Amplicon sequence** | **3'Most** | **Function** |
|---|---|---|---|---|---|---|
| **1** | **EIF3A (EIF3S10)** | 8661 | Hs01025769_m1 | aagctttcaa attctgcctc aatacacgcg taaggctgaa ttccgtaaact gtgtgacaa | No | Control gene |
| **2** | **HADHA** | 3030 | Hs00426191_m1 | gttgccatttcatgccaatacagaatagcaa caaaagacagaaaaacagtattaggta cccctgaagttttgctgggggccttacagga gcaggaggcacacaaaggctgcccaaaa tggtgggtgtgcctgctgtccatctgttctc | No | Control gene |
| **3** | **UBE2D2** | 7322 | Hs00366152_m1 | tgttgtgtgatcccaa tccagatgatcctttagtgcctgagattgctcg gatcta | Yes | Control gene |
| **4** | **EIF5** | 1983 | Hs01028813_g1 | aacgtatcccaccaaatattttggttgtgagc tgggagcacagacccagtttgatgttaaga atgac | No | Control gene |
| **5** | **PPIE** | 10450 | Hs01102333_m1 | gtctactatccatggccaactctggcccaaa caccaatggctctcagttcttcctgacatgtg acaagacagactggctggatggcaagcat gtg | Yes | Control gene |
| **6** | **RPLP0** | 6175 | Hs00420895_gH | aaactgctgcctcatatccgggggaatgtg ggctttgtgttcaccaaggaggacctcactg agatcagggacatgt | Yes | Control gene |

The classifier was built by establishing a set of 10 genes, meeting the criteria of quality and as few features as possible, selected on a full training set using fusion methods with a classifying feature based on the cytological evaluation.

The optimal gene set includes the following 10 genes DCSTAMP, NOD1, SLC26A7, EMP2, EGR1, MPZL2, ITGA2, MET, SLPI, and TPO presented in Table 1.

Gene expression values normalized using a calibrator are entered into the classifier. The result of the Bethesda test of the tested sample is important as it determines the choice of the [B345] or [B26] classifier model. After the procedure, the computer screen displays the result of the tested sample in the form of a boxplot (possibly a mandolin with points or histograms) for the benign and malignant classes, for each gene with the expression value of the sample plotted, and shows the established prediction of the class, the probability of malignancy, and the numerical values of all the features of the sample on the basis of which the classification took place.

The method of distinguishing benign and malignant thyroid nodules using the classifier has been confirmed in an experimental setting, where thyroid nodule samples with an established malignant result have been validated by the postoperative histopathological examination of resected thyroid nodules whereas benign samples in long-term follow-up of the patient.

In the procedure described below, in order to identify genes for differentiating thyroid nodules, modifications have been made to conventional testing methods, and test sets based on classification features, i.e., gene expression and cytological score, are designed to increase the accuracy of predicting the outcome of a thyroid nodule sample.

The study used total RNA isolated from material from a fine-needle biopsy of thyroid nodules taken under ultrasound guidance.

General RNA extraction methods are well known in the field and described in standard molecular biology textbooks. The most common method of RNA extraction is the isolation of total RNA of a cell, while other isolation methods can be used to obtain specific RNA or to conduct, for example, poly(A)RNA isolation. Such an exemplary procedure for processing a biological sample, including isolation of total RNA of a cell, is described in Example 1, which illustrates one possible implementation of the invention.

Gene expression levels can be determined by quantitative RT-PCR (quantitative reverse transcription PCR, RT-qPCR) or other PCR-based methods, as well as microarray-based methods or NGS transcriptome sequencing, or others known in the field or combinations thereof.

### Example 1

Total RNA isolated from a fine-needle biopsy sample of the thyroid nodule taken under ultrasound guidance was used in this analysis.

RNA isolation was performed using Qiagen's miRNeasy micro kit according to the manufacturer's recommendations. RNA concentration was measured with the high-sensitivity Qubit^{™} RNA HS Assay Kit using a Qubit 2.0 fluorometer. For cDNA synthesis, a Sensiscript cDNA reagent kit was used, with Sensiscript Reverse Transcriptase (RT-S), RT buffer, dNTPS mixture, and RNase-free water. Random Nanomers, OligodT(23) primers, and RNase inhibitor at 40 units/µl were also used. The initial RT buffer was diluted to make 1x concentrated buffer, with which the inhibitor was diluted in a 3:1 ratio. 20 ng of total RNA was used for the reaction.

The composition of the cDNA synthesis is summarized in the table below:

| Reagents | Volume (per reaction) | Final concentration |
|---|---|---|
| 10x Buffer RT | 1 µl | 1x |
| dNTP mixture (5nM each) | 1 µl | 0,5 nM each |
| Random Nanomer Primers (50 µM) | 0,8 µl | 2,5 µM |
| OligodT(n23) Primer (40 µM) | 0,25 µl | 1 µM |
| RNAse Inhibitor (10 units/µl) | 0,5 µl | 1 unit |
| RT-S Polymerase (4 units/µl) | 0,5 µl | 0,2 unit |
| RNAse free H₂O | 5,95 µl | |
| Final volume per sample | 10µl | |

An additional pre-amplification step (pre-amplification) is used for RNA samples with low RNA concentrations, allowing real-time PCR reaction without introducing amplification bias.

The pre-amplification step was carried out using TaqMan PreAmp Master Mix reaction mixture and Taqman Preamp Pool gene-specific primer mix. The preamplification reaction was carried out using a QuantStudio 12k Flex system in 96-well plates under the following conditions:
- AmpliTaq Gold DNA polymerase activation - 10 minutes, 95°C
- denaturation - 15 seconds, 95°C
- attachment of primers and chain elongation - 1 minute, 60°C.
- completion of the reaction - 10 minutes, 99°C
14 cycles of amplification were performed. After the reaction, the sample was diluted 20x by adding 190 ul of RNase-free water.

The composition of the cDNA pre-amplification reaction is summarized in table below:

| Reagents | Volume (per reaction) | Final concentration |
|---|---|---|
| 2x Taqman Preamp Master mix | 5 µl | 1x |
| Taqman Preamp Pool | 2,5 µl | |
| cDNA template | 2,5 µl | |
| Final volume | 10 µl | |

In the next step, the reaction mixture, RNase-free water, and preamplified cDNA were applied to the 384-well plate. The plate was centrifuged for 1 min at 2,000 rpm. Next, samples were transferred from the 384-well plate to the OpenArray at the dispensing station. The OpenArray plate was sealed with foil and placed in the press for 10s. After removal, the plate was filled with oil, and the cap was sealed.

The composition of the cDNA amplification reaction is summarized in the table below:

| Reagents | Volume (per reaction) |
|---|---|
| 2x TaqmanOpenarray Real-Time PCR Mastermix | 2,6 µl |
| RNAse-free water | 1,4 µl |
| cDNA template | 1,5 µl |
| Final volume | 5,5 µl |

The qPCR reaction was performed on a QuantStudio 12K FLEX system with an OpenArray block according to the Openarray Taqman Gene Expression protocol (Thermofisher).

Normalization of gene expression measured on the qPCR plate is carried out using a calibrator and six reference genes (Table 2), followed by the analysis of the result, including verification that all reference genes return signal from the quantitative RT-PCR reaction. The condition for correct normalization is that all reference genes return a signal.

After correct normalization, data concerning cytological result and gene expression are entered into the classifier. Based on this data, the classifier determines the probability of malignancy of the thyroid nodule and the nature of the sample as malignant or benign. After the procedure, the computer screen displays the result of the tested sample in the form of a boxplot (possibly a mandolin with points or histograms) for the benign and malignant class of each gene with the expression value of the sample plotted and shows the prediction of the class, the probability of malignancy and the numerical values of all the features of the sample on the basis of which the classification took place.

The gene expression analysis can be carried out using the above-mentioned RT-qPCR method, oligonucleotide microarrays, or RNA-seq transcriptome sequencing (RNA-seq; next-generation sequencing; NGS).

In addition, the above processes can also be used to amplify genes in the sample material containing RNA, fresh or frozen, as well as fixed with fixatives.

### Example 2

This example concerns the sample of the thyroid nodule with cytological result of the Bethesda B4 (suspicious for a follicular neoplasm), which may warrant surgery.

RNA was isolated from the sample, and the expression of a set of genes was measured by RT-qPCR with normalization, and classification was performed.

The sample was classified as benign, with a probability of malignancy of 0.017 (1.7%),

Based on the expanded diagnosis, taking into account the probability of malignancy established by the classifier, it was decided that surgery was not necessary. Instead, close clinical observation is applied. The ongoing observations confirm the benign nature of the thyroid nodule under study and the correct findings of the classifier.

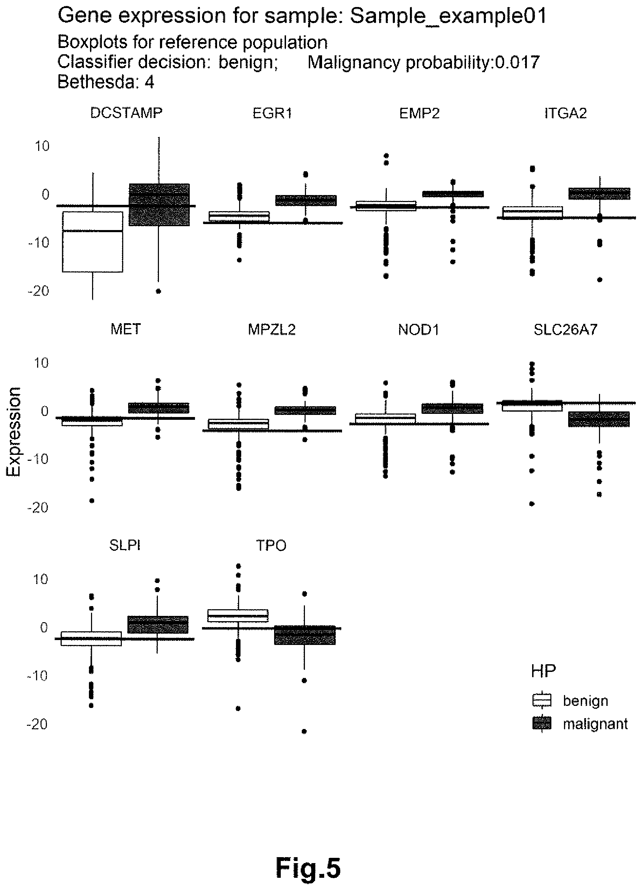

Fig.5 shows with a black horizontal line the expression value for the tested sample against the population used to teach the model.

### Example 3

The thyroid nodule sample was cytologically evaluated as Bethesda category B4 (suspicious for a follicular neoplasm), which may warrant diagnostic surgery.

The expression of a set of genes was measured by the RT-qPCR method, normalization was applied, and classification was performed. The sample was classified as malignant, with a probability of a malignant lesion of 0.977 (97.7%).

Based on the expanded diagnosis, including the results of the molecular examination with the use of the classifier, the decision was made to operate. Post-operative histopathological examination confirmed the diagnosis of thyroid carcinoma

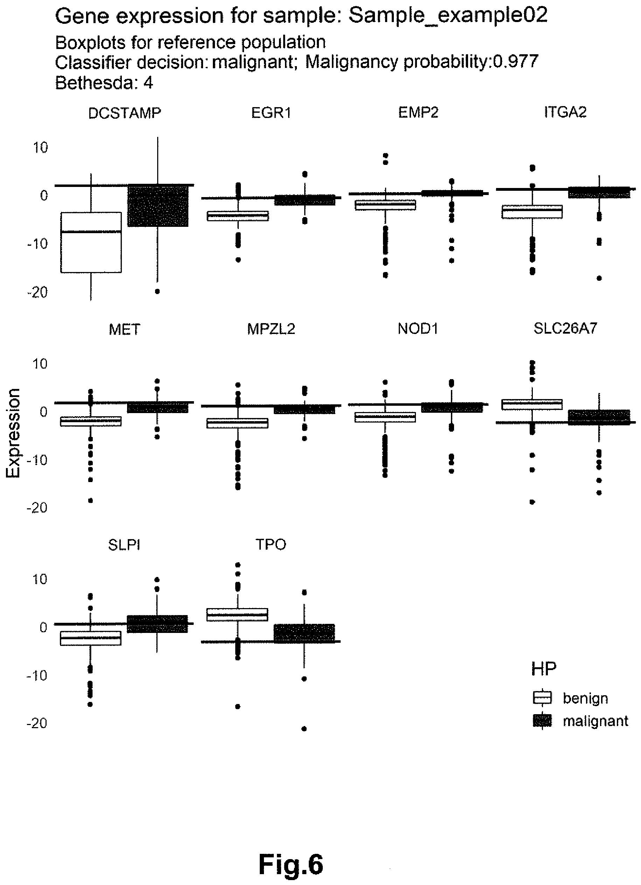

Fig.6 shows with a black horizontal line the expression value for the tested sample against the population used to teach the model.

### Example 4

The example concerns a sample of a thyroid nodule assessed cytologically as Bethesda category B3 (follicular lesion of undetermined significance), which requires expanded diagnostics to determine the nature of the lesion.

RNA was isolated from the sample, and the expression of a set of genes was measured, according to Example 1. The sample was classified as malignant, with a probability of malignancy of 93%.

Further analysis of the biopsy material and verification of the findings revealed an inappropriate initial classification of the sample according to the Bethesda category. In the experts' opinion, the sample should be assigned to category V.

The example illustrates the essential possibility of changing clinical management as a result of the classifier since the risk of malignancy in the Polish patient population for the Bethesda B3 category is estimated at 2% - 5%. In this case, without the supportive diagnosis of the classifier, the patient would be diagnosed with malignant neoplasm too late.

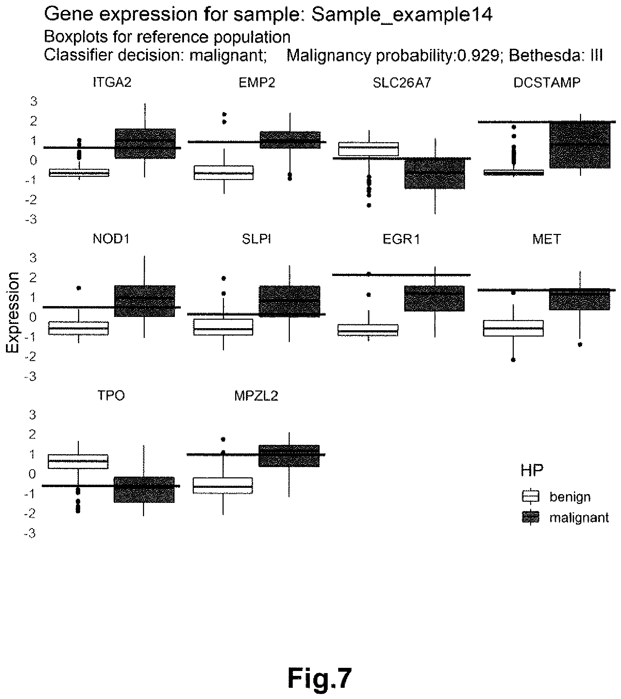

Fig.7 shows with a black horizontal line the expression value for the tested sample against the population used to teach the model.

### Example 5

The example concerns a sample of a thyroid nodule, assessed cytologically as Bethesda category B4 (suspicious for a follicular neoplasm), which may be an indication for diagnostic surgery.

The expression of a set of genes was measured, and the sample was classified according to Example 1.

The sample was classified as benign, with a probability of a malignant lesion of 9.6%. In contrast, the risk of malignancy in a thyroid nodule established for the Bethesda IV category in the Polish population is estimated at 10-30%. Therefore, surgery was deemed unnecessary.

Further analysis of the biopsy material revealed an incorrect initial classification of the sample. In the experts' opinion, the sample should have been assigned to the Bethesda III category (follicular lesion of undetermined significance), which confirms the correctness of the classifier's indications.

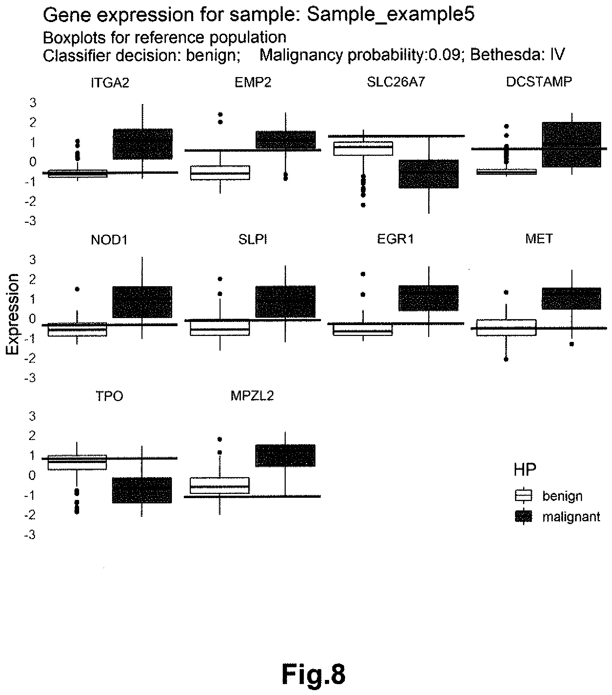

Fig.8 shows with a black horizontal line the expression value for the test sample against the population used to teach the model.

### References:

1.Alexander, Erik K, Giulia C Kennedy, Zubair W Baloch, Edmund S Cibas, Darya Chudova, James Diggans, Lyssa Friedman, et al. 2012. "Preoperative Diagnosis of Benign Thyroid Nodules with Indeterminate Cytology." The New England Journal of Medicine 367 (8): 705-15. https://doi.org/10.1056/NEJMoa1203208.
2.Chudova, Darya, Jonathan I Wilde, Eric T Wang, Hui Wang, Nusrat Rabbee, Camila M Egidio, Jessica Reynolds, et al. 2010. "Molecular Classification of Thyroid Nodules Using High-Dimensionality Genomic Data." The Journal of Clinical Endocrinology and Metabolism 95 (12): 5296-5304. hftps.//doi.org/10.1210/jc.2010-1087.
2.González, Hernán E., José R. Martinez, Sergio Vargas-Salas, Antonieta Solar, Loreto Veliz, Francisco Cruz, Tatiana Arias, et al. 2017. "A 10-Gene Classifier for Indeterminate Thyroid Nodules: Development and Multicenter Accuracy Study." Thyroid 27 (8): 1058-67. https://doi.org/10.1089/thy.2017.0067.
4.Lithwick-Yanai, Gila, Nir Dromi, Alexander Shtabsky, Sara Morgenstern, Yulia Strenov, Meora Feinmesser, Vladimir Kravtsov, et al. 2017. "Multicentre Validation of a MicroRNA-Based Assay for Diagnosing Indeterminate Thyroid Nodules Utilising Fine Needle Aspirate Smears." Journal of Clinical Pathology 70 (6): 500-507. https://doi.org/10.1136/jclinpath-2016-204089.
5. Nikiforov, Yuri E., Sally E. Carty, Simon I. Chiosea, Christopher Coyne, Umamaheswar Duvvuri, Robert L. Ferris, William E. Gooding, et al. 2015. "Impact of the Multi-Gene ThyroSeq Next-Generation Sequencing Assay on Cancer Diagnosis in Thyroid Nodules with Atypia of Undetermined Significance/Follicular Lesion of Undetermined Significance Cytology." Thyroid 25 (11): 1217-23. https://doi.org/10.1089/thy.2015.0305.
6.Zafereo, Mark, Bryan Mclver, Sergio Vargas-Salas, José Miguel Dominguez, David L. Steward, F. Christopher Holsinger, Emad Kandil, et al. 2020. "A Thyroid Genetic Classifier Correctly Predicts Benign Nodules with Indeterminate Cytology: Two Independent, Multicenter, Prospective Validation Trials." Thyroid 30 (5): 704-12. https://doi.org/10.1089/thy.2019.0490.
7.Haugen B, Alexander E, Bible KC, Doherty GM, Mandel SJ,.Nikiforov YE, Pacini F I wsp. 2015 American Thyroid Association Management Guidelines for Adult Patients with Thyroid Nodules and Differentiated Thyroid Cancer: The American Thyroid Association Guidelines Task Force on Thyroid Nodules and Differentiated Thyroid Cancer. Thyroid. 2016 Jan 1; 26(1): 1-133
8.Cibas ES, Ali SZ. The 2017 Bethesda System for Reporting Thyroid Cytopathology. Thyroid. 2017 Nov;27(11):1341-1346
9. Placzek A, Pluciennik A, Kotecka-Blicharz A, Jarzab M and Mrozek D, Bayesian Assessment of Diagnostic Strategy for a Thyroid Nodule Involving a Combination of Clinical Synthetic Features and Molecular Data, IEEE Access, vol. 8, pp. 175125-175139, 2020, doi:10.1109/ACCESS.2020.3026315.

**Table 3**

| **Assay.ID** | **Gene symbol** | **Gene name** | **Gene. Alias(es)** | **RefSeq(s)** | **GenBank. mRNA(s)** | **ENTREZ ID** | **3'.Most** | **Amplicon sequence** |
|---|---|---|---|---|---|---|---|---|
| Hs00229255_m1 | DCSTAMP | dendrocyte expressed seven transmembrane protein | FIND; TM7SF4; hDC-STAMP | XM_017013878.1; XM_011517321.1; NM_030788.3 | BC1 12020.1; AK095417.1; BC069349.1 ; BC112018.1; AF277290.1; AF305068.1 | 81501 | No | aggaactcaatattat ccatgattcttcctttaa tatatctgtgtttgaaccc aactgtatcccaaaacc aaaattccttctatctg agacctgggttcctctc |
| This gene encodes a seven-pass transmembrane protein that is primarily expressed in dendritic cells. The encoded protein is involved in a range of immunolnqical functions carried out by dendritic cells. This protein plays a role in osteoclastopenesis and myeold differentiation. | | | | | | | | |
| Hs00152928_m1 | EGR1 | early growth response 1 | AT225; G0S30; KROX-24; NGFI-A; TIS8; ZIF-268; ZNF225 | NM_001964.2 | KM114058.1; AK298101.1; X52541.1; M62829.1; BCD73983.1; AK301065.1; M80583.1 | 1958 | Yes | cttttcctgacatctctct gaacaacgagaagg tgctggtggagaccag ttaccccagcc aaaccactcgac |
| The protein encoded by this gene belongs to the family o C2H2-type zinc finger EGR proteins. It is a nuclear protein and acts as a transcriptional regulator. The products of the target genes it activates are involved in the processes of differentiation and mitogenesis. Studies suggest that it is a tumor suppressor gene. | | | | | | | | |
| Hs00171315_m1 | EMP2 | epithelial membrane protein 2 | XMP | NM_001424.5; XM_006720864.3 | U52100.1; AK313134.1; SF981157.1; BC009687.1; X94770.1 | 2013 | No | agagtactccacgctg caggcggtccaggcc accatgatcctctcca ccattctctgctgcat cgccttcttcatctt cgtgctccag |
| The gene encodes proteins of the PMP22 / EMP family. The encoded protein regulates cell membrane composition. It is associated with various functions, including endocytosis, cell signaling, cell proliferation, cell migration, cell adhesion, cell death, cholesterol homeostasis, urinary albumin excretion, and embryo implantation. It is known to negatively regulate caveolin-1, a scaffolding protein that is a major component of the cavities in the membrane of caveolae found in most cell types. Through activation of PTK2, it positively regulates vascular endothelial growth factor A. It also modulates the function of specific integrin isomers in the cell membrane. Up-regulation of this gene has been linked to neoplastic progression in a wide variety of tissues. Mutations in this gene are associated with nephrotic syndrome type 10 (NPHS10). | | | | | | | | |
| Hs00158127_m1 | ITGA2 | integrin subunit alpha 2 | BR; CD49B; GPIa; HPA-5; VLA-2; VLAA2 | NM_002203.3; NR_073103.1; NR_073104.1; NR_073105.1; NR_073106.1 | BC143509.1; AK307952.1; BC143508.1; BP280760.1; X17033.1; BC143505.1; BC143511.1 | 3673 | No | attttaaattgttgttt ggcctacaatgttgg tctcccagaagc aaaaatattttccgg tccttcaa |
| The gene encodes a protein that functions in regulating neuronal excitability. The encoded protein forms an M channel through association with the products of related genes KCNQ2 or KCNQ5, which encode integral membrane proteins. Defects in this gene cause benign familial neonatal seizures type 2 (BFNC2). | | | | | | | | |
| Hs01565584_m1 | MET | MET protooncogene, receptor tyrosine kinase | AUTS9; DFNB97; HGFR; RCCP2; c-Met | XM_006715990.2; NM_001127500.2; NM_001324401.1; NM_001324402.1; XM_011516223.1; NM_000245.3 | EU826572.1; EU826573.1; EU826575.1; AB209898.1; EU826574.1; X54559.1; EU826576.1; J02958.1; EU176015.1; EU826567.1; BC130420.1; EU826577.1; EU826568.1; EU826569.1; EU826570.1; EU826579.1; EU826571.1 ; AK296974.1 | 4233 | No | ggttgtggtttctc gatcaggaccat caaccoctatgtg aattttctcctgg actcccatccag tgtctcca |
| It encodes an S-adenosyl-L-methionine-dependent methyltransferase family protein and a methyltransferase-like protein 7B. METTL7B plays a role in lipid metabolism, the development of severe preeclampsia, and tumor progression. Previous studies have indicated that METTL7B may have a dual function in tumor progression. | | | | | | | | |
| Hs01083647_m1 | MPZL2 | myelin protein zero-like 2 | EVA; EVA1 | NM_144765.2; NM_005797.3 | AY359061.1; BC017774.1; AK290326.1; DA163069.1; AF275945.1; AF030455.1; AF304447.1 | 10205 | No | ctctttggcctatagcag ctgtggaaatttatacct cccgggtgctggagg ctgttaatgggacagat gctcggttaaaatg |
| The gene is expressed in the thymus and several epithelial structures early in embryogenesis. Its ability to mediate cell adhesion through homophilic interaction and its selective regulation by T-cell maturation may suggest involvement in the early stages of thymic organogenesis. The protein has a characteristic V-type domain and two potential N-glycosylation sites in the extracellular domain; a putative serine phosphorylation site for casein kinase 2 is also present in the cytoplasmic tail. | | | | | | | | |
| Hs01036720_m1 | NOD1 | nucleotide binding oligomerization domain containing 1 | CARD4; CLR7.1; NLRC1 | XM_011515079.1; XM_005249572.1; NM_006092.2; XM_005249568.1; XM_011515088.2; XM_011515087.1; XM_017011674.1; XM_017011675.1; XM_011515085.1; XM_011515084.1; XM_011515083.1; XM_006715633.2; XM_011515081.2; XM_005249576.1 ; XM_011515080.2 | AF126484.1; AK023969.1; AF113925.1; BC040339.1; KU178514.1 | 10392 | Yes | cagactcagcgt aaaccagatcact gacggtggggt aaaggtgctaag cgaagagctga ccaaatac |
| The gene encodes the pendrin, an anion transporter. Mutations in this gene are associated with Pendred syndrome, an autosomal recessive disease. | | | | | | | | |
| Hs01104163_m1 | SLC26A7 | solute carrier family 26 member 7 | SUT2 | NM_052832.3; NM_001282357.1; NM_001282356.1; NM_134266.1 | AJ413229.2; AF331521.1; BC 114474, 1; AJ413228.1; AJ413230.1; BC113866.1; AK 122933.1 | 115111 | No | gttgaggtttacatgg actgtaaaggcagga gtgtggatgtattgttag cccattgtacagcttc cttgataaaagc aatgacgt |
| This gene is one member of a family of sulfate/anion transporter genes. This gene has abundant and specific expression in the kidney. | | | | | | | | |
| Hs00268204_m1 | SLPI | secretory leukocyte peptidase inhibitor | ALK1; ALP; BLPI; HUSI; HUSI-I; MPI; WAP4; WFDC4 | NM_003064.3 | X04470.1; AF114471.1; AX772818.1; AK312192.1; BC020708.1; X04503.1 | 6590 | No | ttcaaagctggagt ctgtcctcctaag aaatctgcccagtg ccttagatacaag aaacctgagtgccag |
| This gene encodes a secreted inhibitor that protects epithelial tissues from serine proteases. It is found in various secretions, including seminal plasma, cervical mucus, and bronchial secretions, and has an affinity for trypsin, leukocyte elastase, and cathepsin G. its inhibitory effect contributes to the immune response by protecting epithelial surfaces from attack by endogenous proteolytic enzymes. This antimicrobial protein has antibacterial, antifungal, and antiviral activity. | | | | | | | | |
| Hs00892519_m1 | TPO | thyroid peroxidase | MSA; TDH2A; TPX | NM_175719.3; NM_175721.3; NM_175722.3; NM_001206744.1; NM_001206745.1; NM_000547.5; XM_011510380.2; XM_011510381.2; XM_011510379.2; | AB208960.1; X17358.1; AF533528.1; J02970.1; AF439430.1; Y00406.1; DA958481.1; M17755.2; BC085448.1 ; J02969.1 | 7173 | No | gaccaccccagat ggggcgcctcc aacacggccctgg cacgatggctccctcc |
| This gene encodes a membrane-bound glycoprotein. The encoded protein acts as an enzyme and plays a crucia role in thyroid gland function. The protein encoded by TPO is involved in the iodination of tyrosine residues in thyroglobulin and phenoxy-ester formation between pairs of iodinated tyrosines to generate the thyroid hormones thyroxine and triiodothyronine. Mutations in this gene are associated with several disorders of thyroid hormonogenesis, including congenital hypothyroidism, congenital goiter, and thyroid hormone organification defect IIA. | | | | | | | | |

## Claims

1. A method of distinguishing benign and malignant thyroid nodules in the nodule biopsy sample, classified according to the Bethesda system from II to VI category, including
- RNA isolation steps, and
- determination of expression levels for a larger number of genes,
and using the classifier algorithm
- comparing the gene expression of the thyroid nodule sample with the gene expression of the samples in the training dataset and then
- identifying the sample as a malignant or benign and determining the probability of malignancy of the thyroid nodule,
whereby a classifier algorithm with a set of classifier features is trained on a dataset training samples of thyroid nodules with gene expression correlated with the nature of the thyroid nodule, especially benign or malignant tumors, including follicular thyroid carcinoma and papillary thyroid carcinoma,
**characterized in that**
the expression level of a set of the following 10 genes DCSTAMP, NOD1, SLC26A7, EMP2, EGR1, MPZL2, ITGA2, MET, SLPI, TPO is determined,
and the normalization of the expression of each gene of this set is carried out under the endogenous control of at least four normalizing genes among the following EIF3A (EIF3S10), HADHA, UBE2D2, EIF5, PPIE, and RPLP0, most favorably of these six normalizing genes,
and the normalized expression level of a set of genes above or below a certain differential level indicates the malignant nature of the nodule, with at least 90% accuracy for the diagnosis of thyroid nodule malignancy,
whereby the probability of malignancy of a tested sample is determined according to the classifier's decision rule on the basis of classifier features, taking into account the gene expression levels in the training set determined for each sample of this set for the same set of genes and under the same normalization conditions.

2. The method, according to claim 1, **characterized in that** for each sample of the training data set, the set of classifier features includes at least one of the following: cytological assessment, nodule diameter, patient's age and sex, expressed in numerical form or as a synthetic value of the transforming function.

3. The method, according to claim 1, **characterized in that** RT-qPCR, oligonucleotide microarrays, or RNA-seq transcriptome sequencing are used to detect the expression level of a set of genes

4. Use of the set of hybridization probes contained in Table 1, columns 2 and 6 for detecting in a thyroid nodule sample the expression level of a corresponding gene from a set of 10 genes and for determining the nature of the nodule by the method according to claims. 1, using a computer software product having a computer-readable medium and resources for measuring the normalized expression level of a set of genes, performing classification, and determining the probability of malignancy of a nodule sample.
